# EUROPEAN PATENT APPLICATION

(11) **EP 4 393 518 A2**
(43) Date of publication of application: **03.07.2024**
(21) Application number: 24163918.6
(22) Date of filing: 04.07.2017
(51) Int. Cl.: A61L 9/03

(54) **VOLATILE SUBSTANCE EVAPORATION DEVICE**

(30) Priority: 08.07.2016 ES 201630936
(62) Divisional of application: 17737247.1
(71) Applicant: Zobele Holding SpA, 38123 Trento (IT)
(72) Inventor: LUQUE VERA, Sergio, 08019 Barcelona (ES); CABALLERO TAPIA, Moisés, 08019 Barcelona (ES); LLORENTE ALONSO, Joaquim, 08019 Barcelona (ES)
(74) Representative: Herrero & Asociados, S.L.

(57) **Abstract**

The invention relates to a volatile substance evaporation device comprising a body (3) containing a liquid and a heating element heating said liquid for evaporating same, wherein said heating element is a heating film (1). Furthermore, said body (3) defines an evaporation surface, and said heating film (1) preferably has an area substantially the same as the area of said evaporation surface. The invention allows obtaining a homogenous and quick heat distribution.

## Description

The present invention relates to a volatile substance evaporation device for evaporating volatile substances, such as air fresheners or insecticides, comprising a heating element.

### Background of the Invention

Heating elements or thermistors with ceramic or plastic encapsulation are commonly used today for volatile substance evaporators.

Due to inertia, the encapsulated heating elements or thermistors take a long time to reach the required temperature. Their design does not allow a correct heat distribution throughout the surface to be heated and limits the geometry of the devices due to its dimensions.

Furthermore, current resistors are hard to assemble in a casing and require wires for connection.

It must also be indicated that heating films are used on the market for heating surfaces of components in other fields of the art, such as rear-view mirrors in the automotive field or heated floors in the construction field, these films having dimensions and requirements that are very different from volatile substance evaporators.

Therefore, the objective of the present invention is to provide a volatile substance evaporation device in which a homogenous and quick heat distribution can be obtained.

### Description of the Invention

The evaporation device of the invention solves the mentioned drawbacks, having other advantages that will be described below.

The volatile substance evaporation device according to the present comprises a body containing a liquid and a heating element heating said liquid for evaporating same, and is characterized in that said heating element is a heating film.

Advantageously, said body defines an evaporation surface, and said heating film has an area substantially the same as the area of said evaporation surface.

Furthermore, said heating film is preferably a film made of a plastic material provided with at least one printed circuit, said printed circuit being able to be completely or partially activated, if it comprises more than one printed circuit, being able to comprise at least one electronic component, for example a thermistor.

According to two alternative embodiments, said evaporation surface is a face of said body or said evaporation surface is a wick.

According to alternative embodiments, said body can be a pad or a container comprising one or more housings for containing one or more liquids.

Surfaces with the desired geometry can be created with the heating film which, given that it has very little mass, allows reaching the desired temperatures in one tenth of the time required in an encapsulated resistance, and heat is distributed throughout the printed circuit, covering the entire desired surface.

Furthermore, since it is a film, it can be bent in order to be adapted to the surface to be heated, achieving maximum effectiveness regardless of the shape of the surface to be heated. All this means that the user will be able to notice the fragrance or the effect of the insecticide quickly and effectively as a result of the speed at which the correct evaporation temperature and good heat distribution are reached.

Other benefits of this application in the field of volatile substance diffusers is that it allows avoiding the use of wires that are currently used for connecting the resistors, since said diffusers can be attached directly to a connector.

Furthermore, as they relate to printed circuits, electronic elements such as thermistors can be incorporated therein. Since they are located in the heat generation source, the temperature that is applied can be precisely controlled.

Furthermore, the heating film can be coated on at least one of its faces with an adhesive layer, therefore making it easier to fix same in the device.

The heating film can be applied as normal resistors and systems for the self-regulation of temperature. Furthermore, it allows use thereof in low-voltage devices (12 V or less), such as battery-powered devices or devices that are connected to the power outlet of a car; and high voltage (220-230 V) can also be applied directly.

### Brief Description of the Drawings

To better understand what has been set forth, drawings schematically depicting a practical embodiment only by way of a non-limiting example are attached.
Figure 1 is a plan view of the heating film which is used as a heating element in the evaporation device according to the present invention;
Figure 2 is a perspective view of a first embodiment of the evaporation device according to the present invention;
Figure 3 is a perspective view of a second embodiment of the evaporation device according to the present invention;
Figure 4 is a perspective view of a third embodiment of the evaporation device according to the present invention; and
Figure 5 is a perspective view of a fourth embodiment of the evaporation device according to the present invention.

### Description of a Preferred Embodiment

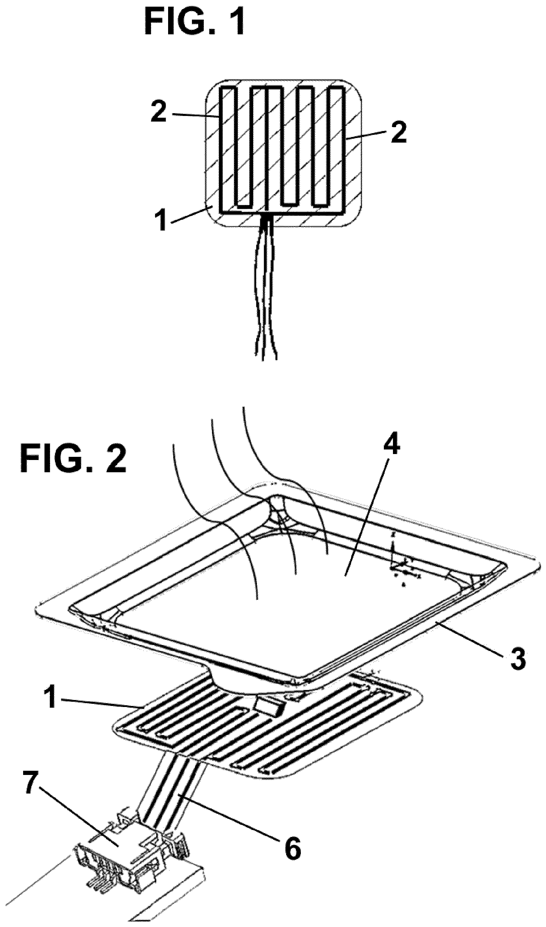

Figure 1 shows a heating film 1 which is used as a heating element in the volatile substance evaporation device according to the present invention.

This heating film 1 is made of a plastic material and includes one or more printed circuits 2, which can be completely or partially activated, and which can include one or more electronic components, for example, a thermistor for controlling and regulating temperature.

For partial activation, the heating film 1 comprises a plurality of independent printed circuits 2, one or more of said printed circuits being able to be activated.

Figure 2 shows a first embodiment of the evaporation device according to the present invention.

In this first embodiment, the device comprises a body 3 in the form of a container provided with a housing 4 for a liquid. As seen in the drawing, the heating film 1 is placed close to one of the faces of the body 3 for heating the liquid and causing the evaporation of the volatile substances.

In this case, the evaporation surface is a membrane, and its area substantially coincides with the area defined by the heating film 1.

The device according to this embodiment is characterized by having a large evaporation surface compared to its size. As a result of using the heating film, any of the surfaces of the body, other than the membrane, can be easily covered regardless of its shape or size.

A homogenous distribution of heat in the liquid inside the housing 4 is therefore achieved, making evaporation of the volatile substances easier.

In this case, the heating film 1 comprises a thermistor integrated therein. As a result of this and the connection to a printed circuit board, the exact temperature being applied to the body can be known.

This allows two functions: applying controlled temperature to the liquid, achieving a constant evaporation; and providing different temperatures to the body and achieving different evaporation intensities.

Another benefit in this case is the integration, by means of a tongue 6, of the contacts required for activating and regulating the heating film 1. This tongue 6 is inserted into a connector 7. This allows eliminating wires and welds, making it easier to assemble the device, since there is no possibility of the wires catching on something, and a device with a very small size can be made.

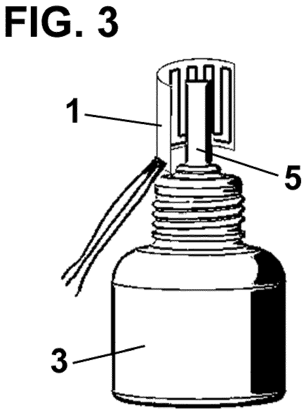

Figure 3 shows a second embodiment of the evaporation device according to the present invention. For the sake of simplicity, in this second embodiment, and in the following embodiments that will be described below, the same reference numbers are used to identify the same or equivalent elements.

In this embodiment, the body 3 is a container provided with a wick 5 as an evaporation surface, around which said heating film 1 is placed. As a result of the flexible nature of the heating film 1, the placement thereof around the wick 5 is very simple.

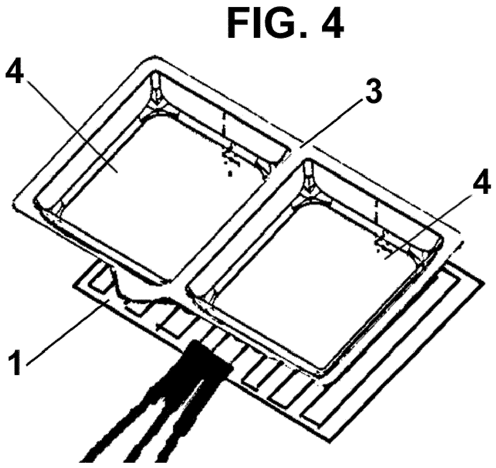

In the third embodiment shown in Figure 4, the body 3 is a container provided with two housings 4 for two different liquids, although it may include more housings. In this case, the heating film 1 will preferably comprise two independent printed circuits 2 for activating the evaporation of either liquid or both liquids at the same time, as desired. Furthermore, a sequence for evaporating both liquids can be programmed as the user desires.

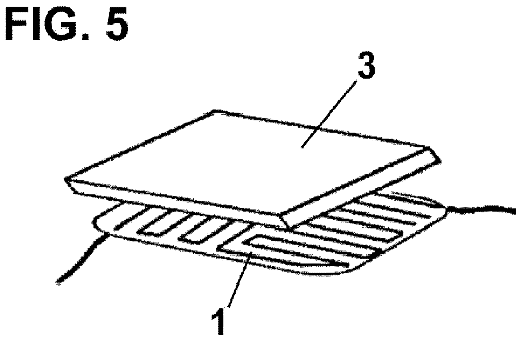

In the fourth embodiment shown in Figure 5, the body 3 is a pad impregnated with a liquid (usually made up of cellulose), the larger surface of which rests on the heating film 1. When activated, the volatile substances are released through the rest of the contact surfaces that are in contact with air.

In this particular case, the heating film 1 is a PTC type and is therefore automatically regulated and does not require any electronics to that end. The heat distribution is the same throughout the entire contact surface.

In this specific embodiment, optimization is observed in terms of device assembly and design, since each of the wires powering the device has the desired position and direction, without the need to place them together or aligned with one another.

Although reference has been made to a specific embodiment of the invention, it is obvious for a person skilled in the art that the evaporation device described is susceptible to a number of variations and modifications, and that all the mentioned details can be replaced with other technically equivalent details without departing from the scope of protection defined by the attached claims.

## Claims

1. Volatile substance evaporation device comprising a body (3) containing a liquid and a heating element heating said liquid for evaporating same, **characterized in that** said heating element is a heating film (1).

2. Volatile substance evaporation device according to claim 1, wherein said body (3) defines an evaporation surface, and said heating film (1) has an area substantially the same as the area of said evaporation surface.

3. Volatile substance evaporation device according to claim 1 or 2, wherein said heating film (1) is a film made of a plastic material provided with at least one printed circuit (2).

4. Volatile substance evaporation device according to any one of the preceding claims, wherein said heating film (1) comprises at least one electronic component, such as a thermistor.

5. Volatile substance evaporation device according to claim 2, wherein said evaporation surface is a face of said body (3).

6. Volatile substance evaporation device according to claim 2, wherein said evaporation surface is a wick (5).

7. Volatile substance evaporation device according to claim 1, wherein said body (3) is a pad.

8. Volatile substance evaporation device according to claim 1, wherein said body (3) is a container comprising one or more housings (4) for containing one or more liquids.

9. Volatile substance evaporation device according to claim 1, wherein said heating film (1) comprises an adhesive layer on at least one of its faces.
